# EUROPEAN PATENT APPLICATION

(11) **EP 3 991 635 A1**
(43) Date of publication of application: **04.05.2022**
(21) Application number: 19935037.2
(22) Date of filing: 28.06.2019
(51) Int. Cl.: A61B 5/00

(54) **MONITORING DEVICE, METHOD FOR SETTING REFERENCE BASELINE AND READABLE STORAGE MEDIUM**

(71) Applicant: Shenzhen Mindray Bio-Medical Electronics Co., Ltd., Shenzhen, Guangdong 518057 (CN)
(72) Inventor: JIA, Yingjie, Shenzhen, Guangdong 518057 (CN); JIANG, Haoyu, Shenzhen, Guangdong 518057 (CN); YE, Wenyu, Shenzhen, Guangdong 518057 (CN); HE, Xianliang, Shenzhen, Guangdong 518057 (CN)
(74) Representative: KIPA AB
(86) International application number: PCT/CN2019/093696
(87) International publication number: WO 2020/258251

(57) **Abstract**

Provided are a monitoring device, a method for setting reference baseline and a readable storage medium, determining whether a system error is introduced into the monitoring device due to at least one event such as the movement of the target object, the change of the monitoring part of the target object, the change of the position of the target object, and the change of the environment in which the target object is located, according to at least one of the moving state of the target object, the connection state of the target object and the signal acquisition device (1), the environmental information where the target object is located, and the analysis result of the monitoring device, and when the system error is introduced, the current reference baseline of the monitoring device is updated according to the preset rules, and then the monitoring is continued based on the new reference baseline. In this way, the current reference baseline of the monitoring device can be adjusted in time when the system error is introduced, so as to avoid errors in the analysis result of the monitoring device, false alarms and missed alarms caused by the introduction of the system error, improving the accuracy of the monitoring device.

## Description

### TECHNICAL FIELD

The disclosure relates to the technical field of medical devices, and more particularly to a monitoring device, a method for setting reference baseline and a readable storage medium.

### BACKGROUND

Monitoring devices are commonly operable to measure and display information that is about physiological parameter values, such as a heart rate, blood oxygen and blood pressure of a target object, as well as the characteristic waveform shape of each physiological signal, and to give an alarm when the above information is abnormal or has an obvious trend of change, thus providing the basis of the emergency treatment and therapy for the medical staff to minimize complications, alleviate and eliminate the disease condition. Accordingly, they are increasingly widely used.

The monitoring device acquires signals from the monitored part of the target object in real time, analyzes the acquired signals at regular intervals, and then compares the analyzed results with the stored reference value (i.e. reference baseline) as the analysis benchmark to obtain the trend of change of the information that is about the physiological parameter values and the characteristic waveform shape of each physiological signal, and finally gives a corresponding alarm when the information that is about physiological parameter values and the characteristic waveform shape of each physiological signal, is abnormal or has an obvious trend of change.

However, in the practical applications, some system errors may also lead to large changes in the signal waveform, thus resulting in the false alarm or missed alarm, such that the accuracy of the monitoring device is reduced.

### SUMMARY

This disclosure mainly provides a monitoring device, a method for setting reference baseline and a readable storage medium for avoiding false alarms and missed alarms caused by system errors and improving the accuracy of the monitoring device.

According to a first aspect, a monitoring device is provided in one embodiment of the present disclosure, which includes a signal acquisition apparatus, a state detection apparatus and a signal processing apparatus; wherein
the signal acquisition apparatus is operable to connect with a target object and acquire a physiological signal of a monitored part of the target object;
the state detection apparatus is operable to detect state information of the target object, wherein the state information reflects at least one of the following states of the target object: a movement state, a connection state between the target object and the signal acquisition apparatus, and environment information of the target object; and
the signal processing apparatus, which is respectively connected with the signal acquisition apparatus and the state detection apparatus, is operable to analyze the physiological signal acquired by the signal acquisition apparatus, obtain physiological information of the target object; compare the physiological information with a current reference baseline of the monitoring device to obtain an analysis result of the monitoring device; determine whether the monitoring device has a system error according to the state information detected by the state detection apparatus and the analysis result of the monitoring device; and update the current reference baseline according to a preset rule when the monitoring device has the system error .

According to a second aspect, a method for setting reference baseline is provided in one embodiment of the present disclosure, which includes:
acquiring state information of a target object and a physiological signal of a monitored part of the target object, wherein the state information reflects at least one of the following states of the target object: a movement state, a connection state between the target object and a signal acquisition apparatus, and environment information of the target object;
analyzing the acquired physiological signal to obtain physiological information of the target object;
comparing the physiological information with a current reference baseline of the monitoring device to obtain an analysis result of the monitoring device;
determining whether the monitoring device has a system error according to the state information and the analysis result of the monitoring device; and
updating the current reference baseline according to a preset rule when the monitoring device has the system error.

According to a third aspect, a computer-readable storage medium is provided in one embodiment of the present disclosure, which includes a program that can be executed by a processor to implement the method described above.

The monitoring device, method for setting reference baseline and readable storage medium according to the above embodiments, can determine whether the monitoring device has the system error into the monitoring device due to events, such as the movement of the target object, the change of the monitored part of the target object, the change of the body position of the target object and the change of the environment of the target object according to the analysis result of the monitoring device and at least one of: the movement state of the target object, the connection state between the target object and the signal acquisition apparatus, the environment information of the target object; and can update the current reference baseline according to the preset rule when the monitoring device has the system error . In this way, the current reference baseline of the monitoring device can be adjusted in time when the monitoring device has the system error, so as to avoid the analysis result error, the false alarm and missed alarm of the monitoring device which are caused by the system errors, thus improving the accuracy of the monitoring device.

### BRIEF DESCRIPTION OF THE DRAWINGS

FIG. 1 is a structural diagram of a monitoring device according to an embodiment of this disclosure.
FIG. 2 is a specific structural diagram of a monitoring device according to an embodiment of this disclosure.
FIG. 3 is a frame diagram of a monitoring device networking system according to an embodiment of this disclosure.
FIG. 4 is a structural diagram of a monitoring device according to a specific embodiment of this disclosure.
FIG. 5 is a flowchart of a method for setting reference baseline according to an embodiment of this disclosure.
FIG. 6 is a flowchart of a method for setting reference baseline according to a specific embodiment of this disclosure.
FIG. 7 is a flowchart of a method for determining whether a monitoring device has a system error into the monitoring device in a specific embodiment of this disclosure.
FIG. 8 is a schematic diagram of a reference baseline setting interface according to an embodiment of this disclosure.

### DESCRIPTION OF THE PREFERRED EMBODIMENTS

The disclosure will be further described in detail below through specific embodiments in combination with the accompanying drawings. The expression "connection" and "linkage" mentioned in this disclosure include both direct and indirect connection (linkage), unless otherwise specified.

When using the monitoring device to monitor the physiological state of the target object, the monitoring device analysis technology can be operable to compare the physiological characteristics at multiple different time points before and after, detect the trend of change of the information that is about the physiological parameter values and the characteristic waveform shape of each physiological signal. In order to implement the monitoring device analysis smoothly, intuitively display the trend of change over time of the information that is about the physiological parameter values and the characteristic waveform shape of each physiological signal, and more accurately determine the development process of diseases, such as acute myocardial infarction, it is necessary to accurately select the reference baseline according to the individual target object and specific monitoring scene, and to accurately select the alarming value of the information that is about the physiological parameter values (such as heart rate, blood pressure, blood oxygen, St offset amplitude, QT interval and so on) and the characteristic waveform shape of each physiological signal of the monitored object for the monitoring device analysis and alarming.

In the practical application, the reference baseline is generally fixed, that is, the reference baseline is preset according to clinical experience and remains unchanged throughout the monitoring process. However, this method cannot exclude the impact of individual differences the target object and environmental conditions of the target object on the monitoring process. Such impact is significantly larger, when events, such as the change of the movement state of the target object, the change of the monitored part of the target object, the change of the body position of the target object and the change of the environment light source, occur. For example, in the ECG monitoring process, if the lead of the monitoring device falls off and then is reconnected, the monitored part will generally deviate from that before the reconnection. When such position deviation is large, even if the cardiac physiological state of the target object itself does not change significantly, its cardiac waveform shape may also have a large steady-state jump. For another example, it is difficult for the target object to maintain a fixed posture for a long time in the process of long-term monitoring. If its posture changes, it may also bring in interfere to the monitoring of parameters such as the blood pressure and blood oxygen.

Therefore, if the reference baseline cannot be updated in time in this case, the monitoring device will have the systematic comparison error, resulting in the false alarm or missed alarm of the monitoring device.

Based on this, the technical scheme of this disclosure is proposed. In the embodiment of this disclosure, the physiological information of the target object at different time points is compared with the current reference baseline of the monitoring device to obtain the analysis result of the monitoring device. Then according to the analysis result of the monitoring device and the detected state information of the target object, whether there is a system error which is caused by at least one event, is determined. Wherein the at least one event comprises the movement of the target object, the change of the monitored part of the target object, the change of the body position of the target object and the environment condition which comprises the change of the external light source. When there is a system error, the current reference baseline is updated according to a preset rule.

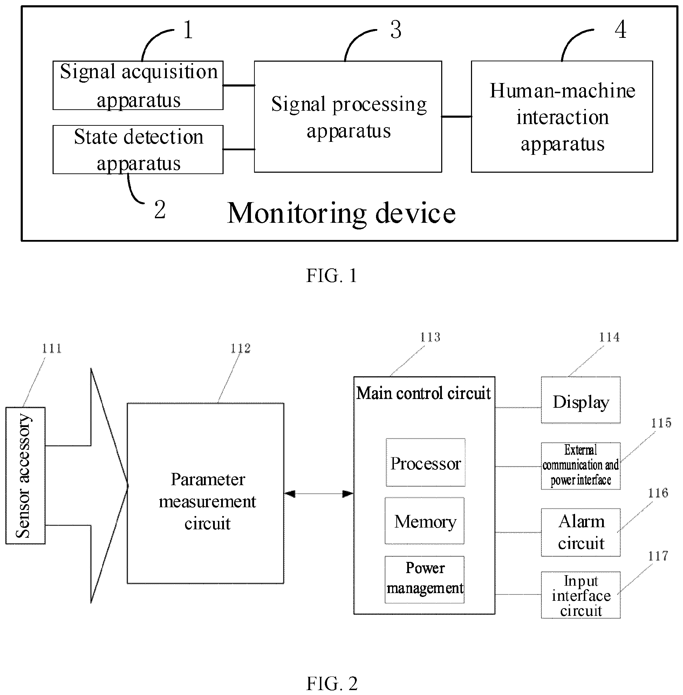

The embodiment of this disclosure provides a monitoring device, whose structural diagram is shown in FIG. 1. The monitoring device includes a signal acquisition apparatus 1, a state detection apparatus 2 and a signal processing apparatus 3. The signal acquisition apparatus 1 is operable to connect with a target object and acquire a physiological signal of a monitored part of the target object. The state detection apparatus 2 is operable to detect state information of the target object. Wherein the state information reflects at least one of the following states of the target object: a movement state, a connection state between the target object and the signal acquisition apparatus, and environment information of the target object. The movement state includes a motion state of the target object and/or a change of body position of the target object. For example, the state detection apparatus 2 may be at least one of: an acceleration sensor for detecting motion state information of the target object, a displacement sensor for detecting body position information of the target object to determine whether the body position of the target object changes, a falling-off detection apparatus which is connected with the signal acquisition apparatus 1 for detecting the connection state between the signal acquisition apparatus 1 and the target object and an imaging apparatus for detecting environment information of the environment where the target object is located (such as an intensity of an environmental light source, a type of the environmental light source, etc.). Wherein the acceleration sensor and the displacement sensor are operable to connect with the target object. The connection state between the signal acquisition apparatus 1 and the target object includes that the signal acquisition apparatus 1 is connected with the target object or the signal acquisition apparatus 1 is disconnected from the target object. The state detection apparatus 2 can obtain image information and/or acceleration velocity signals reflecting the motion state of the target object, the monitored part of the target object, the body position of the target object, etc. The signal processing apparatus 3 is respectively connected with the signal acquisition apparatus 1 and the state detection apparatus 2. The signal processing apparatus 3 is operable to analyze the physiological signal acquired by the signal acquisition apparatus 1, and to obtain physiological information of the target object. Wherein the physiological information of the target object includes measured value of the physiological parameter, a characteristic waveform shape of the physiological signal and other information of the target object. The signal processing apparatus 3 is further operable to compare the physiological information which includes measured value of the physiological parameter, a characteristic waveform shape of the physiological signal and other information of the target object, with a current reference baseline of the monitoring device to obtain an analysis result of the monitoring device. Then the signal processing apparatus 3 is further operable to update the current reference baseline according to a preset rule for avoiding a false alarm or missed alarm of the monitoring device when the signal processing apparatus 3 determines that the monitoring device has a system error according to the analysis result of the monitoring device and the state information detected by the state detection apparatus 2, such as an image and acceleration sensor. Wherein the system error is caused by at least one event which includes a motion of the target object, a change of the monitored part of the target object, a change of a body position of the target object and a change of external light source and so on.

In the practical application, the monitoring device can also include a human-machine interaction apparatus 4, which is connected with the signal processing apparatus 3 for detecting input information of a user and outputting the detection result of the target object.

Referring to FIG. 2 in combination with FIG. 1, a specific structural diagram of a monitoring device is provided. The signal acquisition apparatus 1 at least includes a parameter measurement circuit 112. The parameter measurement circuit 112 includes at least one parameter measurement circuit selected from an ECG parameter measurement circuit, a respiratory parameter measurement circuit, a body temperature parameter measurement circuit, a blood oxygen parameter measurement circuit, a noninvasive blood pressure parameter measurement circuit, an invasive blood pressure parameter measurement circuit, and so on. Each parameter measurement circuit 112 is respectively connected with a sensor accessory 111 externally inserted through a corresponding sensor interface. The sensor accessory 111 includes at least one of detection accessories corresponding to the detection of the physiological parameters such as ECG, respiration, blood oxygen, blood pressure and body temperature. The parameter measurement circuit 112 is mainly operable to connect the sensor accessory 111 to obtain acquired physiological parameter signal and may include at least two measurement circuits for the physiological parameters. The parameter measurement circuit 112 may be, but is not limited to, a physiological parameter measurement circuit (module), a human physiological parameter measurement circuit (module) or a sensor for acquiring human physiological parameters. Specifically, the parameter measurement circuit 112 obtains a physiological sampling signal related to a patient from an external physiological parameter sensor accessory by means of an extended interface, and then obtains physiological data after processing the physiological sampling signal for alarming and displaying. The extended interface can also be operable to output a control signal which is about how to acquire the physiological parameter and is outputted by a main control circuit to an external physiological parameter monitoring accessory through a corresponding interface, so as to control the monitoring of the physiological parameters of the patient.

The signal processing apparatus 3 may include a main control circuit 113, which needs to include at least one processor and at least one memory. Of course, the main control circuit 113 may also include at least one of a power management module, a power IP module, an interface conversion circuit, etc. The power management module is operable to control the power-on and power-off operation of the whole machine, a power-on time sequence of each power domain inside a board card and battery charging and discharging, etc. The power IP module is a separate power module firmed by associating a principle diagram of a power supply circuit unit that is frequently and repeatedly invoked, with a PCB layout. That is, an input voltage is converted into an output voltage by means of a predetermined circuit, where the input voltage and the output voltage are different. For example, the voltage of 15 V is converted into 1.8 V, 3.3 V, 3.8 V, etc. It can be understood that the power IP module may be single-channel or multi-channel. If the power IP module is single-channel, the power IP module may convert one input voltage into one output voltage. If the power IP module is multi-channel, the power IP module may convert one input voltage into a plurality of output voltages, and voltage values of the plurality of output voltages may be the same or different, such that different voltage requirements of a plurality of electronic components can be met at the same time. In addition, the module has few external interfaces, and works in the system as a black box decoupled from an external hardware system, which improves the reliability of the entire power system. The interface conversion circuit is operable to convert a signal output by a master control minimum system module (i.e., at least one processor and at least one memory in the main control circuit 113) into an input standard signal required to be received by an actual external device. For example, supporting an external VGA display function is to convert an RGB digital signal output by a master control CPU into a VGA analog signal, and supporting an external network function is to convert an RMII signal into a standard network differential signal.

In addition, the monitoring device may also include one or more of a local display 114, an alarm circuit 116, an input interface circuit 117, and an external communication and power interface 115. The main control circuit 113 is operable to coordinate and control each board card, circuit and device in a multi-parameter monitoring device or the module assembly. In this embodiment, the main control circuit 113 is operable to control data interaction and control signal transmission between the parameter measurement circuit 112 and a communication interface circuit, and transmit the physiological data to the display 114 for display, or may receive a user control instruction entered from a touchscreen or a physical input interface such as a keyboard and keys, and may also output a control signal for acquiring the physiological parameters. The alarm circuit 116 may be an audible and visual alarm circuit. The main control circuit 113 completes the calculation of the physiological parameters, and may send calculation results and waveforms of the parameters to the host (such as a host with a display, a PC, and a central station and so on) by means of the external communication and power interface 115. The external communication and power interface 115 may be one or a combination of local area network interfaces composed of Ethernet, a token ring, a token bus, and an optical fiber distributed data interface (FDDI) as the backbone of these three networks, or may be one or a combination of wireless interfaces such as infrared, Bluetooth, WIFI, and WMTS communication, or may be one or a combination of wired data connection interfaces such as RS232 and USB. The external communication and power interface 115 may also be one of a wireless data transmission interface and a wired data transmission interface or a combination thereof. The host may be any computing device, such as a host of the monitoring device, an electrocardiograph machine, an ultrasonic diagnosis instrument, and a computer, and can form a monitoring device once installed with matching software. The host may alternatively be a communication device such as a mobile phone, and the multi-parameter monitoring device or the module assembly sends, by means of a Bluetooth interface, data to the mobile phone supporting Bluetooth communication, so as to implement remote transmission of data.

The multi-parameter monitoring device assembly may be arranged outside a housing of the monitoring device as an independent externally inserted parameter module, may be inserted into the host (including a main control board) of the monitoring device to form a plug-in monitoring device as a part of the monitoring device , or may be connected to the host (including the main control board) of the monitoring device by means of a cable, and the externally inserted parameter module is used as an external accessory of the monitoring device . Alternatively, the parameter processing module may also be built in the housing to be integrated with a main control module, or physically separated and arranged in the housing to form an integrated monitoring device.

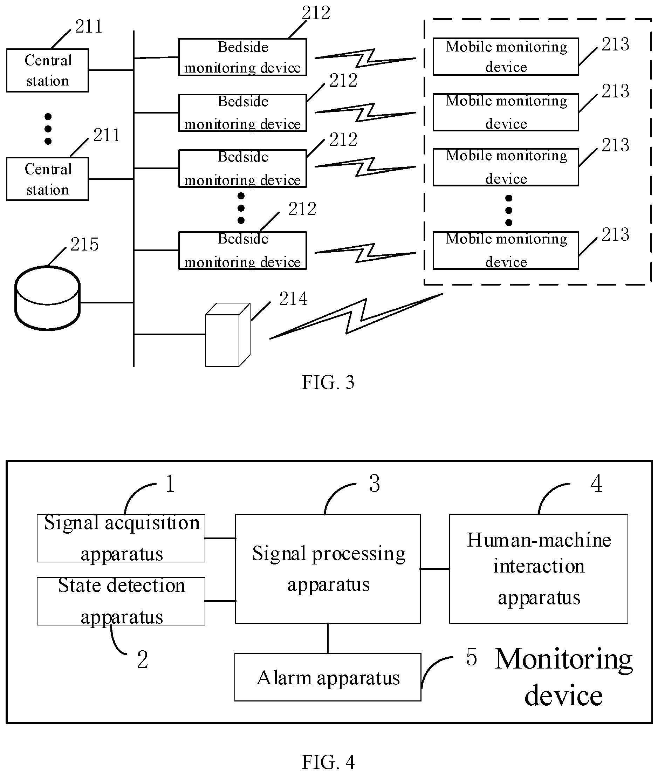

As shown in FIG. 3, a monitoring device networking system for use in a hospital is provided. By using the monitoring device networking system, the data of a monitoring device may be saved as a whole, where patient information and nursing information can be managed centrally and may be stored in association, which facilitates the storage of historical data and associated alarming. In the system shown in FIG. 3, a bedside monitoring device 212 may be provided for each hospital bed. In addition, each bedside monitoring device 212 may further be paired with a mobile monitoring device 213 for transmission. The mobile monitoring device 213 provides a simple and portable multi-parameter monitoring device or module assembly and can be worn on the body of a patient for mobile monitoring of the patient. With the wired or wireless communication between the mobile monitoring device 213 and the bedside monitoring device 212, physiological data generated during the mobile monitoring process may be transmitted to the bedside monitoring device 212 for display, or transmitted through the bedside monitoring device 212 to a central station 211 for a doctor or nurse to check, or transmitted through the bedside monitoring device 212 to a data server 215 for storage. In addition, the mobile monitoring device 213 may further directly transmit, through a wireless network node 214 arranged in the hospital, the physiological data generated during the mobile monitoring process to the central station 211 for storage and display, or transmit, through the wireless network node 214 arranged in the hospital, the physiological data generated during the mobile monitoring process to the data server 215 for storage. It can be seen that, the data corresponding to the physiological parameter displayed on the bedside monitoring device 212 may originate from the sensor accessory directly connected to the monitoring device, or from the mobile monitoring device 213, or from the data server.

In practical application, the signal processing apparatus 3 is also operable to determine whether the physiological information is a first valid value after the monitoring device is turned on after obtaining the physiological information of the target object, such as measured values of the physiological parameters and characteristic waveform shapes of the physiological signal. If the physiological information is the first valid value after the monitoring device is turned on, then an instruction for displaying a reference baseline setting interface is sent to the human-machine interaction apparatus 4 for displaying and instructing the user to set the physiological information as the reference baseline. If the user chooses to confirm on the initial reference baseline setting interface that the physiological information is set as the reference baseline, the human-machine interaction apparatus 4 generates a confirmation instruction and sends the confirmation instruction to the signal processing apparatus 3. When the signal processing apparatus 3 receives the confirmation instruction, it sets the first valid value as the reference baseline. If the user does not confirm or cancels the setting on the initial reference baseline setting interface, the human-machine interaction apparatus 4 generates a cancellation instruction and sends the cancellation instruction to the signal processing apparatus 3. When the signal processing apparatus 3 receives the cancellation instruction, it uses the reference baseline based on the clinical reference value built in the monitoring device. That is, the preset reference value is set as the reference baseline. Then, the physiological information of the target object, such as measured values of the physiological parameters and characteristic waveform shapes of the physiological signal are continuously acquired again. In another embodiment, the signal processing apparatus 3 can also automatically set the physiological information as the reference baseline when it is determined that the physiological information is the first valid value after the monitoring device is turned on.

In a specific embodiment, in the real-time measurement process of the target object by the monitoring device, the signal processing apparatus 3 determines whether there is a system error which is caused by at least one event comprising a movement of the target object, a change of the monitored part of the target object, a change of a body position of the target object and an environment condition which comprises a change of the external light source, based on the analysis result of the monitoring device and the detected state information of the target object, the specific implementation is as follows. At first, the signal processing apparatus 3 determines whether the state information of the target object changes, if yes, the signal processing apparatus 3 enters a state where a baseline needs to be confirmed. Or else, the signal processing apparatus 3 continue to determine whether the monitoring device is currently in the state where a baseline needs to be confirmed due to the previous change of the state information. When the monitoring device is in the state where a baseline needs to be confirmed, the signal processing apparatus 3 determines whether the monitoring device has a system error according to the analysis result of the monitoring device. When the state information of the target object changes, the monitoring device may have the system error. At this time, the monitoring device is set to the state where a baseline needs to be confirmed, and whether the monitoring device has the system error is determined in the state where a baseline needs to be confirmed according to the analysis result of the monitoring device. Specifically, in the state where a baseline needs to be confirmed, the signal processing apparatus 3 determines whether the analysis results of the monitoring device for consecutive N times all indicate that the physiological information, such as the measured value of the physiological parameters, the characteristic waveform shape of the physiological signals, and so on, are stable. If the analysis results of the monitoring device for consecutive N times all indicate that the physiological information is stable and there is no mutation again, then it is determined that the change of the physiological information of the target object at the time of entering the state where a baseline needs to be confirmed, is due to the system error resulted by at least one event, such as the movement of the target object, the change of the monitored part of the target object, the change of the body position of the target object and the change of the environment light source of the target object and so on. At this time, the reference baseline needs to be adjusted. If the analysis result of the monitoring device indicates that the physiological information is still changing, it is determined that the change of the physiological information of the target object at the time of entering the state where a baseline needs to be confirmed, is due to the physiological state of the target object's heart and other organs. At this time, the monitoring device will exit the state where a baseline needs to be confirmed without adjusting the reference baseline. Wherein N is an integer greater than or equal to 2. The physiological information can include the measured value of the physiological parameter, the characteristic waveform shape of physiological signal and other information. It can be understood that consecutive N times can be calculated by counting the times of analysis.

When determining that the monitoring device is not in the state where a baseline needs to be confirmed, the signal processing apparatus 3 can determine whether there is interference, such as the movement of the target object, the change of the monitored part of the target object, the change of the body position of the target object and so on. If yes, the monitoring device is set to the state where a baseline needs to be confirmed, and the counter is zero cleared to start counting for the N times again. Otherwise, the state of the monitoring device remains unchanged.

When determining that there is a system error, the signal processing apparatus 3 inquiries about a reference baseline setting mode, and directly updates the current reference baseline when the reference baseline setting mode is an automatic mode. When the reference baseline setting mode is a semi-automatic mode, the signal processing apparatus 3 sends an instruction for displaying a reference baseline setting interface to the human-machine interaction apparatus 4. At this time, the human-machine interaction apparatus 4 detects an operation selected by the user to change the reference baseline on the reference baseline setting interface, generates an instruction to change the reference baseline according to the operation, and then sends an instruction to change the reference baseline to the signal processing apparatus 3. When receiving the instruction to change the reference baseline sent by the human-machine interaction apparatus 4, the signal processing apparatus 3 updates the current reference baseline. Specifically, the signal processing apparatus 3 can acquire stable physiological information, such as stable measured value of the physiological parameter, characteristic waveform shape of the physiological signal and other information of the target object after the system error is over and update the current reference baseline to the stable physiological information. Wherein, the stable measured value of the physiological parameter, characteristic waveform shape of the physiological signal and other information can be the measured value of the physiological parameter, characteristic waveform shape of the physiological signal and other information which are in a stable state for a preset time period. In practical application, a time period can be preset. If the measured value of the physiological parameter, characteristic waveform shape of the physiological signal and other information do not change suddenly or exceed a preset change threshold within this time period, it can be considered that the measured value of the physiological parameter, characteristic waveform shape of the physiological signal and other information have reached the stable state.

In a specific embodiment, the state detection apparatus 2 is, for example, a falling-off detection apparatus, which is respectively connected with the signal acquisition apparatus 1 and the signal processing apparatus 3 to detect a connection state between the signal acquisition apparatus 1 and the target object, and to send the connection state to the signal processing apparatus 3. The connection state includes that the signal acquisition apparatus 1 is connected with the target object or the signal acquisition apparatus 1 is disconnected from the target object. In this embodiment, the signal processing apparatus 3 is specifically operable to determine whether the signal acquisition apparatus 1 is disconnected and then reconnected with the target object according to the connection state sent by the falling off detection device 1. If the signal acquisition apparatus 1 is disconnected and reconnected with the target object, the signal processing apparatus 3 sets the monitoring device to the state where a baseline needs to be confirmed. Then, in the state where a baseline needs to be confirmed, the signal processing apparatus 3 determines whether the monitoring device has a system error due to the disconnection and reconnection of the signal acquisition apparatus 1 with the target object according to the analysis result of the monitoring device.

In the practical application, the current reference baseline and/or updated reference baseline can both be displayed on the reference baseline setting interface, and their respective monitoring results can also be displayed based on the current reference baseline and updated reference baseline for comparison and analysis. Further, when it is determined that there is a system error, corresponding indication information can be displayed on the reference baseline setting interface. The indication information is operable to indicate at least one item of the following content: system error state information, cause of system error, parameter status changes, mutated parameter and reference baseline reset. For example, words such as "parameter change may be caused by interference" and "system error due to lead falling off" can be displayed to indicate the user. The indication information can also be in different forms, such as sound, light, voice and vibration, as long as it can indicate the user for the system error, and its specific form is not limited.

After updating the current reference baseline, the signal processing apparatus 3 continues to monitor the physiological information of the target object based on the updated reference baseline. That is, the signal processing apparatus 3 continues to analyze the physiological signal acquired by the signal acquisition apparatus 1 to obtain the physiological information of the target object, such as measured values of the physiological parameters, characteristic waveform shapes of the physiological signal and so on, and then compare the physiological information of the target object, such as measured values of the physiological parameters, characteristic waveform shapes of the physiological signal and so on with the updated reference baseline to obtain the analysis result of the monitoring device, and finally output a monitoring alarm information according to the analysis result. And on this basis, the signal processing apparatus 3 further continues to determine whether there is a system error. When there is a system error, the signal processing apparatus 3 continues to update the current reference baseline according to the preset rules, so as to continuously monitor the target object.

Based on the monitoring device shown in Fig. 1, in a specific embodiment, the monitoring device also includes an alarm apparatus 5 connected with the signal processing apparatus 3. FIG. 4 has shown the structure of the monitoring device, in which the signal processing apparatus 3 is further operable to generate an alarm instruction when the physiological information of the target object, such as measured values of the physiological parameters, characteristic waveform shapes of the physiological signal and so on, changes dynamically relative to the current reference baseline of the monitoring device, but there is no system error (that is, the change of the physiological information is caused by the change of the physiological factors of the target object itself), and send the alarm instruction to the alarm apparatus 5. The alarm apparatus 5 gives an alarm when receiving the alarm instruction. The alarm apparatus 5 may be, for example, a buzzer and/or an indicator lamp. In the present embodiment, it may be the alarm circuit 116 in Fig. 2. It can be understood that the human-machine interaction apparatus 4 can also be operable to display corresponding alarm information when receiving the alarm instructi on.

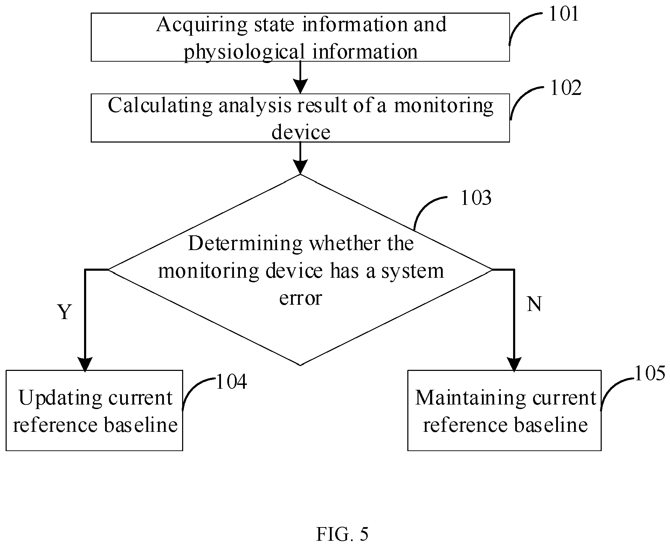

Based on the monitoring device of the above embodiment, the embodiment of this disclosure provides a method for setting a reference baseline. FIG.5 has shown its flowchart. The method can include the following steps.

In step 101, state information and physiological information are acquired.

During the monitoring process of the physiological state of the target object by the monitoring device, the signal acquisition apparatus 1 acquires a physiological signal of the monitored part of the target object in real time, and sends the physiological signal to the signal processing apparatus 3. The signal processing apparatus 3 analyzes the acquired physiological signal to obtain the physiological information of the target object, such as measured values of the physiological parameters, characteristic waveform shapes of the physiological signal and so on. At the same time, the state detection apparatus 1 of the monitoring device detects the state information of the target object, which is used to reflect a movement state of the target object (including a motion state of the target object and/or a change of body position of the target object), a connection state between the target object and the signal acquisition apparatus 1 (including connection and disconnection) and/or environment information of the target object (such as an intensity of an environmental light source, a type of the environmental light source, etc.), and then sends the detected state information to the signal processing apparatus 3.

In step 102, an analysis result of a monitoring device is calculated.

After receiving the physiological information of the target object, such as measured values of the physiological parameters, characteristic waveform shapes of the physiological signal and so on, the signal processing apparatus 3 compares the physiological information with a current reference baseline of the monitoring device to obtain the analysis result of the monitoring device. The analysis result can reflect a change state of the physiological information, such as measured values of the physiological parameters, characteristic waveform shapes of the physiological signal and so on.

In step S103, whether the monitoring device has a system error is determined.

After obtaining the analysis result of the monitoring device and the state information of the target object, the signal processing apparatus 3 determines whether the systematic error caused by at least one event, such as a motion of the target object, a change of the monitored part of the target object, a change of a body position of the target object and a change of external light source and so on, is introduced according to the analysis result of the monitoring device and the state information of the target object. If yes, the method proceeds to step 104, otherwise, to step 105.

In step 104, a current reference baseline is updated.

When determining that the monitoring device has the system error, the signal processing apparatus 3 updates the current reference baseline according to a preset rule. Specifically, the signal processing apparatus 3 inquiries about a reference baseline setting mode, and directly updates the current reference baseline when the reference baseline setting mode is an automatic mode. When the reference baseline setting mode is a semi-automatic mode, the signal processing apparatus 3 sends an instruction for displaying a reference baseline setting interface to the human-machine interaction apparatus 4 and update the current reference baseline when receiving an instruction to change the reference baseline sent by the human-machine interaction apparatus 4. After updating the current reference baseline, the signal processing apparatus 3 can repeat step 101 and subsequent steps based on the updated reference baseline.

In step 105, the current reference baseline is maintained.

When determine that the monitoring device has no system error, the signal processing apparatus 3 keeps the current reference baseline unchanged.

The monitoring device and the method for setting the reference baseline provided by the embodiment of this disclosure, can determine whether the monitoring device has the system error into the monitoring device due to at least one event, such as the movement of the target object, the change of the monitored part of the target object, the change of the body position of the target object and the change of the environment of the target object, according to the analysis result of the monitoring device and the state information of the target object, and can update the current reference baseline according to the preset rule when the monitoring device has the system error. In this way, the current reference baseline of the monitoring device can be adjusted in time when the systematic deviation presents, so as to avoid the false alarm and missed alarm of the monitoring device.

In the embodiment of this disclosure, the systematic deviation (i.e., system error) can take place due to at least one event, such as a change of the connection position between the signal acquisition apparatus 1 and the target object, a change of the external light source, a change of the body position of the target object, etc. The physiological information of the target object can be ECG information, EEG information, etc. Among them, the ECG information can include at least one of QRS waveform shape, ST-T waveform shape, P-wave electrical axis and ST offset.

The following is a detailed description by taking ECG information as the physiological information and taking an ECG lead system (hereinafter referred to as lead) as the corresponding signal acquisition apparatus 1. The ECG lead system can support 3-lead, 5-lead, 6-lead and 12-lead acquisition modes and so on, and its sampling rate and signal amplitude resolution can be set according to the conventional ECG monitoring, such as 1000 Hzh and 250 LSB / mV.

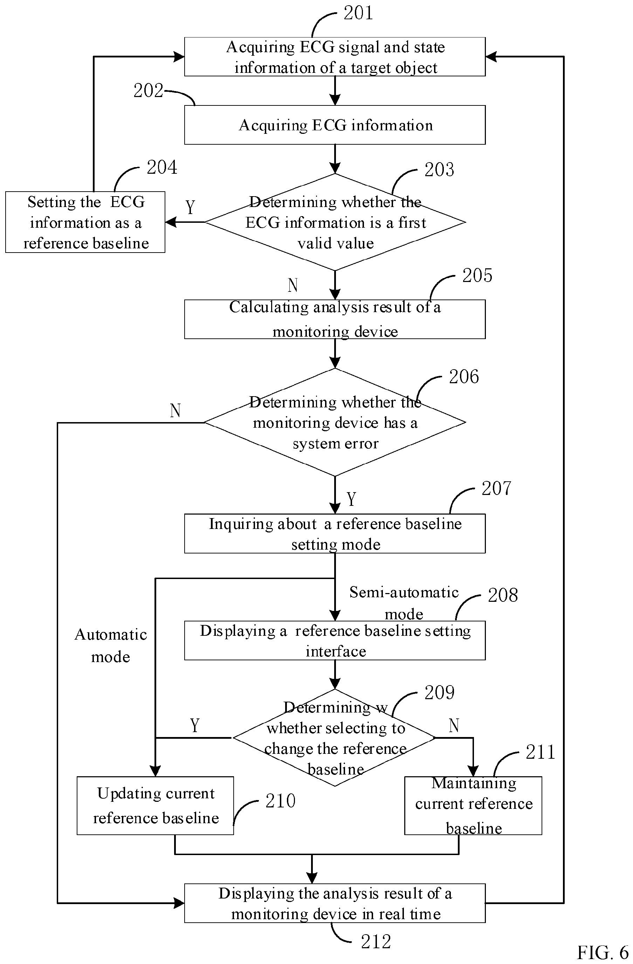

FIG. 6 is a flowchart of a method for setting reference baseline according to a specific embodiment of this disclosure. This method can include the following steps.

In step 201, an ECG signal and state information of a target object are acquired.

After the monitoring device is started, the ECG lead system acquires an ECG signal of a monitored part of the target object in real time and sends the acquired ECG signal to the signal processing apparatus 3. At the same time, the state detection apparatus 2 detects at least one kind of state information, such as a movement state, monitored part, body position and environmental light source of the target object and so on, and sends obtained state information to the signal processing apparatus 3. In practical application, the information obtained by the state detection apparatus 2 may be information such as image and/or acceleration velocity, and so on.

In step 201, ECG information is acquired.

After receiving the ECG signal sent by the ECG lead system, the signal processing apparatus 3 analyzes the ECG signal every other time period (i.e., a time cycle), that is, the signal processing apparatus 3 extracts ECG features to obtain the ECG information of the target object.

In step 203, whether the ECG information is a first valid value is determined.

After obtaining the ECG information of the target object, the signal processing apparatus 3 determines whether the ECG information is the first valid value after the monitoring device is started. If the ECG information is the first valid value after the monitoring device is started, the method proceeds to step 204. Otherwise, the method proceeds to step 205.

In step 204, the ECG information is set as a reference baseline.

If the signal processing apparatus 3 determines that the ECG information is the first valid value after the monitoring device is started, it sets the ECG information as the reference baseline of the monitoring device and returns to step 201 to acquire the ECG information of the target object again. At this time, a baseline setting state of the monitoring device is a state where a baseline needs to be confirmed.

In another embodiment, if the signal processing apparatus 3 determines that the ECG information is the first valid value after the monitoring device is started, the user can determine whether to set the ECG information as the reference baseline of the monitoring device. Specifically, when the signal processing apparatus 3 determines that the ECG information is the first valid value after the monitoring device is started, an initial reference baseline setting interface is displayed on a display interface of the human-machine interaction apparatus 4. The initial reference baseline setting interface can include indication information for indicating the user that whether to set the ECG information as the reference baseline, and a "confirm" operation item. When the user selects the "confirm" operation item, the signal processing apparatus 3 sets the first valid value as the reference baseline, otherwise sets a preset reference value as the reference baseline. Wherein the preset reference value can be a reference baseline based on the clinical reference value.

In step 205, an analysis result of a monitoring device is calculated.

If the signal processing apparatus 3 determines that the ECG information of the target object is a valid value, but still not the first valid value after the monitoring device is started, it compares the ECG information with the current reference baseline of the monitoring device to obtain the analysis result of the monitoring device, which can reflect a trend of change of the ECG information of the target object.

In step S206, whether the monitoring device has a system error is determined.

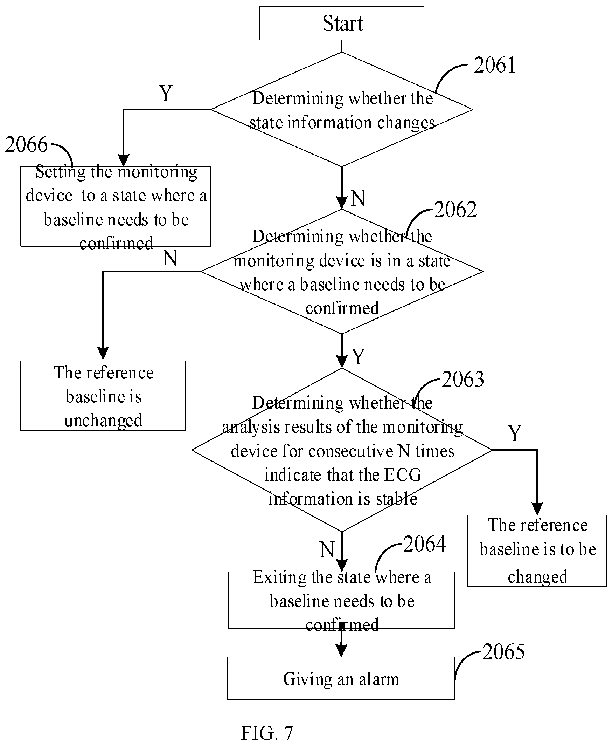

After obtaining the analysis result of the monitoring device, the signal processing apparatus 3 determines whether the monitoring device has a system error according to the analysis result of the monitoring device and the state information of the target object. When the monitoring device has no system error, the analysis result of the monitoring device is displayed in real time, that is, step 212 is performed. Specifically, FIG. 7 shows the flowchart of determining whether the monitoring device has a system error. The signal processing apparatus 3 can determine according to the method steps of FIG. 7. The method can include the following steps 2061 to 2068.

In step 2061, whether the state information changes, is determined.

After obtaining the analysis result of the monitoring device, the signal processing apparatus 3 determines whether there is at least one event, such as a motion of the target object, a change of the monitored part of the target object, a change of a body position of the target object and a change of external light source and so on, according to the state information (such as an image, an acceleration velocity and other state information) obtained by the state detection apparatus 2. If the signal processing apparatus 3 determines that there is at least one of the above events, it considers that the monitoring device is suspected to have the system error and further confirmation is required. Then the method proceeds to step 2066. Otherwise, the method proceeds to step 2062.

In step 2062, whether the monitoring device is in a state where a baseline needs to be confirmed, is determined.

If the signal processing apparatus 3 determines that the state information has not changed, it needs to continue to determine whether the monitoring device is currently in the state where a baseline needs to be confirmed due to the previous change of the state information. If the signal processing apparatus 3 determines that the monitoring device is in the state where a baseline needs to be confirmed, it determines whether the monitoring device has the system error according to the analysis result of the monitoring device, and specifically performs steps 2063 to 2065. Otherwise, the signal processing apparatus 3 keeps the current reference baseline unchanged and returns back to step 2061.

In step 2063, whether the analysis results of the monitoring device for consecutive N times indicate that the ECG information is stable, is determined.

When the signal processing apparatus 3 determines that the monitoring device is in the state where a baseline needs to be confirmed, it then determines whether the analysis results for consecutive N times indicate that the ECG information is stable, in which N is an integer greater than or equal to 2. If the signal processing apparatus 3 determines that the analysis results for consecutive N times indicate that the ECG information is stable, it determines that the change of the ECG information of the target object at the time point of entering the state where a baseline needs to be confirmed is due to the system error caused by the occurrence of at least one event, such as the movement of the target object, the change of the monitored part of the target object, the change of the body position of the target object and the change of the environment light source of the target object and so on. At this time, the reference baseline needs to be modified. In this case, it can be seen that, after the occurrence of at least one event, such as the movement of the target object, the change of the monitored part of the target object, the change of the body position of the target object and the change of the environment light source of the target object and so on, the ECG information quickly reaches a stable state. At this time, the ECG information reaching the stable state can be used as a new reference baseline and the state where a baseline needs to be confirmed is exited at the same time.

If the signal processing apparatus 3 determines that the analysis result of the monitoring device indicates that the ECG information is still changing, it is considered that the change of the ECG information is caused by the physiological state of the target object itself, rather than by at least one system error, such as the movement of the target object, the change of the monitored part of the target object, the change of the body position of the target object and the change of the environment light source of the target object and so on. At this time, the signal processing apparatus 3 performs step 2064 and step 2065, wherein step 2064 and step 2065 are not limited to be performed in order, and they can be performed at the same time.

In step 2064, the state where a baseline needs to be confirmed is exited.

When the signal processing apparatus 3 determines that the analysis result of the monitoring device indicates that the ECG information is still changing, the monitoring device will exit the state where a baseline needs to be confirmed and the current reference baseline remains unchanged.

In step 2065, an alarm is given.

When the signal processing apparatus 3 determines that the analysis result of the monitoring device indicates that the ECG information is still changing, it determines that the change of ECG information is caused by the change of physiological factors of the target object's own heart, and then generates an alarm instruction, and sends the alarm instruction to the alarm device 5. The alarm device 5 alarms when receiving the alarm instruction.

In step 2066, the monitoring device is set to the state where a baseline needs to be confirmed.

When the signal processing apparatus 3 determines that the state information of the target object changes, that is, it is determined that there is at least one interference factor, such as the movement of the target object, the change of the monitored part of the target object, the change of the body position of the target object and the change of the environmental light source and so on, it is considered that the monitoring device may have a system error and further confirmation is needed. At this time, the signal processing apparatus 3 sets the monitoring device to the state where a baseline needs to be confirmed, clears the counter and starts counting for the N times again. When the signal processing apparatus 3 determines whether the monitoring device in the state where a baseline needs to be confirmed has a system error according to the analysis result of the monitoring device, the specific steps may refer to the method from step 2063 to step 2065.

It can be seen from the method of FIG. 7 that, when it is determined that there are events, such as the movement of the target object, the change of the monitored part of the target object and/or the change of the body position of the target object and so on, the monitoring device will enter the state where a baseline needs to be confirmed. In the state where a baseline needs to be confirmed, if it is found that the ECG information is changing all the time, it is considered that the change of the ECG information of the target object at the time point of entering the state where a baseline needs to be confirmed is caused by the physiological factors of the target object's heart. At this time, exit the state where a baseline needs to be confirmed and give an alarm. If it is found that the analysis results from several consecutive times suggest that the ECG information will not change, it is considered that the sudden change of the ECG information of the target object at the time point of entering the state where a baseline needs to be confirmed is caused by the system error difference introduced by at least one event, such as the movement of the target object, the change of the monitored position of the target object, the change of the body position of the target object and the change of the environmental light source, and so on.

If the signal processing apparatus 3 determines that the change of ECG information of the target object is caused by at least one interference factor, such as the movement of the target object, the change of the monitored position of the target object, the change of the body position of the target object and the change of the environmental light source, and so on, according to the method of FIG. 7, that is, it is determined that the monitoring device has a system error, the method proceeds to step 207. Otherwise, the method proceeds directly to step 212.

In step S207, a reference baseline setting mode is inquired about.

When the signal processing apparatus 3 determines that there is a system error, in this embodiment, is corresponding to determining there is a system error when "reference baseline is to be modified" in FIG. 7. At this time, a reference baseline setting mode of the monitoring device is inquired about. If the reference baseline setting mode is a semi-automatic mode, step 208 is performed. If the reference baseline setting mode is an automatic mode, the method proceeds directly to step 210.

In step 208, a reference baseline setting interface is displayed.

When the signal processing apparatus 3 finds that the reference baseline setting mode is the semi-automatic mode, it sends the reference baseline setting interface to the human-machine interaction apparatus 4 for display. Through this interface, the user is instructed to modify the reference baseline, and the user decides whether to modify the current reference baseline.

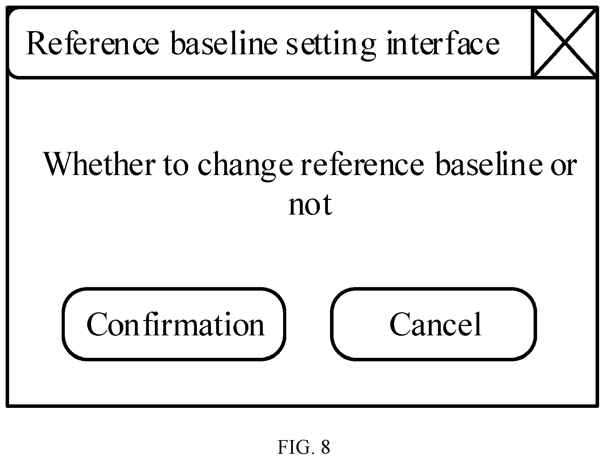

FIG. 8 shows a reference baseline setting interface, on which instruction information such as "whether to change the reference baseline?" can be displayed, and selection keys for "changing the reference baseline" and "not changing the reference baseline" can be provided to the user, which keys can be a "Conformation" function key and a "Cancel" function key respectively.

In step 209, whether selecting to change the reference baseline is determined.

After the signal processing apparatus 3 sends the reference baseline setting interface to the human-machine interaction apparatus 4 for display, the human-machine interaction apparatus 4 detects whether the user selects the operation of changing the reference baseline on the reference baseline setting interface. When the human-machine interaction apparatus 4 detects that the user selects the operation of changing the reference baseline on the reference baseline setting interface, it generates an instruction to change the reference baseline according to the operation, and then sends the instruction to change the reference baseline to the signal processing apparatus 3. When receiving the instruction to change the reference baseline sent by the human-machine interaction apparatus 4, the signal processing apparatus 3 updates the current reference baseline. At this time, the signal processing apparatus 3 determines that the user has selected to change the reference baseline on the reference baseline setting interface, and then performs step 210. When the human-machine interaction apparatus 4 does not detect that the user selects the operation of changing the reference baseline within a certain time, or detects that the user selects the operation of not changing the reference baseline on the reference baseline setting interface, it generates an instruction to cancel the change of the reference baseline according to the operation, and sends the instruction to cancel the change of the reference baseline to the signal processing apparatus 3. At this time, the signal processing apparatus 3 determines that the user does not need to change the reference baseline and then performs step 211.

For example, on the interface shown in FIG. 8, when the user needs to update the current reference baseline, he/she can click the "Confirmation" function key. At this time, the signal processing apparatus 3 will update the current reference baseline. If the user clicks the "Cancel" function key or directly turns off the interface, the signal processing apparatus 3 will maintain the current reference baseline at this time.

In practical application, the reference baseline setting interface can also display the current reference baseline and/or updated reference baseline, as well as the ECG parameter and/or ECG waveform based on the current reference baseline and/or updated reference baseline respectively, so as to facilitate the user to compare. Of course, instruction information can also be displayed on the reference baseline setting interface. For example, words, such as "the change of the ECG information may indicate that there is a system error" can be displayed to the user.

In step S210, the current reference baseline is updated.

When the signal processing apparatus 3 inquiries about that the reference baseline setting mode is the automatic mode, or determines that the user has selected to change the reference baseline on the reference baseline setting interface, the current reference baseline is updated. Specifically, the signal processing apparatus 3 can update the current reference baseline to the ECG information stored when the monitoring device is set to the state where a baseline needs to be confirmed. Alternatively, the signal processing apparatus 3 can also obtain the stable ECG information after the occurrence of the system error, and then update the current reference baseline of the monitoring device to the stable ECG information. The stable ECG information can be the ECG information that does not change within a preset time period.

It can be understood that when the ECG information of the target object changes due to at least one event, such as the movement of the target object, the change of the connection position after the falling off and reconnection of the lead, the change of the body position of the target object and the change of environmental light source, the reference baseline of the monitoring device can be adjusted timely to the stable ECG information of the target object after the movement of the target object, the stable ECG information of the target object after the change of the connection position, the stable ECG information of the target object after the falling off and reconnection of the lead, the stable ECG information of the target object after the change of the body position of the target object, and/or the stable ECG information of the target object after the change of environmental light source, and so on. In such a way, the systematic comparison error caused by the monitoring device which still uses the initial reference baseline for the analysis of the monitoring device, can be avoided.

In step 211, the current reference baseline remains unchanged.

When the signal processing apparatus 3 determines that the user does not need to change the reference baseline, the current reference baseline remains unchanged.

After performing step 210 or step 211, the signal processing apparatus 3 performs step 212 based on the updated reference baseline or the maintained reference baseline.

In step 212, the analysis results of the monitoring device are displayed in real time.

The signal processing apparatus 3 sends the real-time analysis result to the human-machine interaction apparatus 4 for display. After that, the signal processing apparatus 3 continues to monitor the ECG information of the target object, that is, the signal processing apparatus 3 repeats step 201 and subsequent steps.

The method for setting reference baseline according to the above embodiments, can determine whether the monitoring device has the system error into the monitoring device due to at least one event, such as the movement of the target object, the change of the monitored part of the target object, the change of the body position of the target object and the change of the environment of the target object and so on, according to the state information, such as the image and/or acceleration velocity and so on, and the analysis result of the monitoring device; and can update the current reference baseline according to the preset rule when the monitoring device has the system error. In this way, on one hand, the current reference baseline of the monitoring device can be directly updated when determining that the monitoring device has the system error into due to at least one event, such as the movement of the target object, the change of the monitored part of the target object, the change of the body position of the target object and the change of the environment of the target object and so on. On the other hand, the user can choose whether to update the current reference baseline or not when determining that the monitoring device has the system error. In such a way, the intelligent setting of the reference baseline of the monitoring device is realized, and the current reference baseline of the monitoring device can be adjusted to the accurate position in time, so as to avoid the false alarm and missed alarm, thus improving the accuracy of the monitoring device.

The description has been made with reference to various exemplary embodiments herein. However, those skilled in the art will recognize that changes and modifications can be made to the exemplary embodiments without departing from the scope herein. For example, various operation steps and components for performing operation steps may be implemented in different ways according to a specific application or considering any number of cost functions associated with the operation of the system (for example, one or more steps may be deleted, modified, or incorporated into other steps).

In addition, as understood by those skilled in the art, the principles herein may be reflected in a computer program product on a computer-readable storage medium that is pre-installed with computer-readable program codes. Any tangible, non-transitory computer-readable storage medium can be used, including magnetic storage devices (hard disks, floppy disks, etc.), optical storage devices (CD-ROM, DVD, Blu Ray disks, etc.), flash memory, and/or the like. These computer program instructions can be loaded onto a general-purpose computer, a dedicated computer, or other programmable data processing device to form a machine, so that these instructions executed on a computer or other programmable data processing apparatus can generate an apparatus that implements a specified function. These computer program instructions can also be stored in a computer-readable memory that can instruct a computer or other programmable data processing device to operate in a specific manner, so that the instructions stored in the computer-readable memory can form a manufactured product, including an implementation apparatus that implements a specified function. The computer program instructions can also be loaded onto a computer or other programmable data processing device, so that a series of operating steps are performed on the computer or other programmable device to produce a computer-implemented process, so that the instructions executed on a computer or other programmable data processing device can provide steps for implementing specified functions.

Although the principles herein have been shown in various embodiments, many modifications of structures, arrangements, proportions, elements, materials and components particularly suitable for the specific environmental and operational requirements may be used without departing from the principles and scope of the present disclosure. The above modifications and other changes or amendments will be included in the scope of this disclosure.

The foregoing specific description has been described with reference to various embodiments. However, those skilled in the art will recognize that various modifications and changes can be made without departing from the scope of the present disclosure. Therefore, consideration of the present disclosure will be in an illustrative rather than a restrictive sense, and all such modifications will be included within the scope thereof. Also, the advantages of various embodiments, other advantages, and the solutions to problems have been described above. However, the benefits, advantages, solutions to problems, and any elements that can produce these, or solutions that make them more explicit, should not be interpreted as critical, necessary, or essential. The term "comprising", and any other variants thereof used herein are non-exclusive, so that the process, method, document, or device that includes a list of elements includes not only these elements, but also other elements that are not explicitly listed or do not belong to the process, method, system, document, or device. Furthermore, the term "coupling" and any other variations thereof used herein refer to physical connection, electrical connection, magnetic connection, optical connection, communication connection, functional connection, and/or any other connection.

It should be noted that a person of ordinary skill in the art could also make several variations and improvements without departing from the concept of this disclosure, and these variations and improvements would all fall within the scope of protection of this disclosure. Therefore, the protection scope of this disclosure should be in accordance with the appended claims.

## Claims

1. A monitoring device, **characterised in that**, comprising a signal acquisition apparatus, a state detection apparatus and a signal processing apparatus; wherein
the signal acquisition apparatus is operable to connect with a target object and acquire a physiological signal of a monitored part of the target object;
the state detection apparatus is operable to detect state information of the target object, wherein the state information reflects at least one of following states of the target object: a movement state, a connection state between the target object and the signal acquisition apparatus, and environment information of the target object; and
the signal processing apparatus, which is respectively connected with the signal acquisition apparatus and the state detection apparatus, is operable to analyze the physiological signal acquired by the signal acquisition apparatus, obtain physiological information of the target object; compare the physiological information with a current reference baseline of the monitoring device to obtain an analysis result of the monitoring device; determine whether the monitoring device has a system error according to the state information detected by the state detection apparatus and the analysis result of the monitoring device; and update the current reference baseline according to a preset rule when the monitoring device has the system error.

2. The monitoring device according to claim 1, **characterised in that**, in order to determine whether the monitoring device has a system error according to the state information detected by the state detection apparatus and the analysis result of the monitoring device, the signal processing apparatus is specifically operable to:
set the monitoring device to a state where a baseline needs to be confirmed and determine whether the monitoring device has the system error according to the analysis result of the monitoring device, when the state information changes; and
determine whether the monitoring device has the system error according to the analysis result of the monitoring device, when the state information does not change and the monitoring device is in the state where a baseline needs to be confirmed.

3. The monitoring device according to claim 2, **characterised in that**, in order to determine whether the monitoring device has the system error according to the analysis result of the monitoring device in the state where a baseline needs to be confirmed, the signal processing apparatus is specifically operable to:
in the state where a baseline needs to be confirmed, determine that the monitoring device has the system error if analysis results of the monitoring device for consecutive N times all indicate that the physiological information is stable, wherein N is an integer greater than or equal to 2;
wherein the monitoring device exits the state where a baseline needs to be confirmed if the analysis results of the monitoring device for consecutive N times indicate that the physiological information still changes.

4. The monitoring device according to claim 3, **characterised in that**, the monitoring device further comprises an alarm apparatus which is connected with the signal acquisition apparatus;
wherein the signal acquisition apparatus is further operable to generate an alarm instruction when the analysis results of the monitoring device for consecutive N times indicate that the physiological information still changes, and send the alarm instruction to the alarm apparatus; and
the alarm apparatus is operable to give an alarm when receiving the alarm instructi on.

5. The monitoring device according to claim 1, **characterised in that**, after updating the current reference baseline, the signal processing apparatus is further operable to continue to analyze the physiological signal acquired by the signal acquisition apparatus to obtain the physiological information of the target object, compare the physiological information of the target object with the updated reference baseline to obtain the analysis result of the monitoring device, and continue to determine whether the monitoring device has the system error according to the analysis result.

6. The monitoring device according to claim 1, **characterised in that**, the monitoring device further comprises a human-machine interaction apparatus which is connected with the signal processing apparatus and operable to detect input information of a user and output a detection result of the target object.

7. The monitoring device according to claim 6, **characterised in that**, in order to update the current reference baseline according to a preset rule, the signal processing apparatus is specifically operable to:
inquire about a reference baseline setting mode;
directly update the current reference baseline when the reference baseline setting mode is an automatic mode; and
send an instruction for displaying a reference baseline setting interface to the human-machine interaction apparatus and update the current reference baseline when receiving an instruction to change the reference baseline sent by the human-machine interaction apparatus, when the reference baseline setting mode is a semi-automatic mode;
wherein the human-machine interaction apparatus is further operable to detect an operation selected by the user to change the reference baseline on the reference baseline setting interface, generate the instruction to change the reference baseline according to the operation, and then send the instruction to change the reference baseline to the signal processing apparatus.

8. The monitoring device according to claim 7, **characterised in that**, in order to update the current reference baseline, the signal processing apparatus is specifically operable to acquire stable physiological information after the system error is over and update the current reference baseline to the stable physiological information.

9. The monitoring device according to claim 7, **characterised in that**, the reference baseline setting interface is further operable to display the current reference baseline and/or the updated reference baseline.

10. The monitoring device according to claim 7, **characterised in that**, the reference baseline setting interface is further operable to display information for indicating whether there is the system error.

11. The monitoring device according to claim 6, **characterised in that**, the signal processing apparatus is further operable to:
determine whether the physiological information is a first valid value after the monitoring device is started;
send an instruction for displaying a reference baseline setting interface to the human-machine interaction apparatus when the physiological information is the first valid value after the monitoring device is started; and
set the first valid value as the reference baseline when receiving a confirmation instruction from the human-machine interaction apparatus, or set a preset reference value as the reference baseline when receiving a cancellation instruction from the human-machine interaction apparatus.

12. The monitoring device according to claim 1, **characterised in that**, the signal processing apparatus is further operable to:
determine whether the physiological information is a first valid value after the monitoring device is started; and
set the first valid value as the reference baseline when the physiological information is the first valid value after the monitoring device is started.

13. The monitoring device according to claim 1, **characterised in that**, the movement state comprises a motion state of the target object and/or a change of body position of the target object;
wherein the state detection apparatus comprises at least one of: an acceleration sensor, a displacement sensor, a falling-off detection apparatus and an imaging apparatus;
wherein the acceleration sensor, which is connected with the target object, is operable to detect motion state information of the target object and send the motion state information to the signal processing apparatus;
the displacement sensor, which is connected with the target object, is operable to detect body position information of the target object and send the body position information to the signal processing apparatus;
the falling-off detection apparatus, which is connected with the signal acquisition apparatus, is operable to detect the connection state between the signal acquisition apparatus and the target object, and send the connection state to the signal processing apparatus, wherein the connection state includes that the signal acquisition apparatus is connected with the target object or the signal acquisition apparatus is disconnected from the target object; and
the imaging apparatus is operable to detect the environment information of the target object and send the environment information to the signal processing apparatus.

14. The monitoring device according to claim 1, **characterised in that**, the signal acquisition apparatus comprises an ECG lead system.

15. The monitoring device according to claim 14, **characterised in that**, the physiological information includes ECG information, and the ECG information includes at least one of: QRS waveform shape, ST-T waveform shape, P-wave electrical axis, and ST offset.

16. A method for setting reference baseline, **characterised in that**, comprising:
acquiring state information of a target object and a physiological signal of a monitored part of the target object, wherein the state information reflects at least one of the following states of the target object: a movement state, a connection state between the target object and a signal acquisition apparatus, and environment information of the target object;
analyzing the acquired physiological signal to obtain physiological information of the target object;
comparing the physiological information with a current reference baseline of a monitoring device to obtain an analysis result of the monitoring device;
determining whether the monitoring device has a system error according to the state information and the analysis result; and
updating the current reference baseline according to a preset rule when the monitoring device has the system error.

17. The method according to claim 16, **characterised in that**, determining whether the monitoring device has a system error according to the state information and the analysis result, comprises:
setting the monitoring device to a state where a baseline needs to be confirmed and determining whether the monitoring device has the system error according to the analysis result of the monitoring device in the state where a baseline needs to be confirmed, when the state information changes; and
determining whether the monitoring device is in the state where a baseline needs to be confirmed when the state information does not change, and further determining whether the monitoring device has the system error according to the analysis result of the monitoring device when the monitoring device is in the state where a baseline needs to be confirmed.

18. The method according to claim 17, **characterised in that**, determining whether the monitoring device has the system error according to the analysis result of the monitoring device, comprises:
determining the monitoring device has the system error if analysis results of the monitoring device for consecutive N times all indicate that the physiological information does not change, wherein N is an integer greater than or equal to 2; and
enabling the monitoring device to exit the state where a baseline needs to be confirmed if the analysis results of the monitoring device for consecutive N times indicate that the physiological information still changes.

19. The method according to claim 18, **characterised in that**, the method further comprises:
giving an alarm when the analysis results of the monitoring device for consecutive N times indicate that the physiological information still changes.

20. The method according to claim 16, **characterised in that**, after updating the current reference baseline, the method further comprises:
continuing to analyze the acquired physiological signal to obtain the physiological information of the target object, and compare the physiological information of the target object with the updated current reference baseline to obtain the analysis result of the monitoring device; and
continuing to determine whether the monitoring device has the system error according to the analysis result.

21. The method according to claim 16, **characterised in that**, updating the current reference baseline according to a preset rule, comprises:
inquiring about a reference baseline setting mode;
directly updating the current reference baseline, when the reference baseline setting mode is an automatic mode; and
displaying a reference baseline setting interface and determining whether a user selects to change the reference baseline on the reference baseline setting interface and updating the current reference baseline when the user selects to change the reference baseline, when the reference baseline setting mode is a semi-automatic mode.

22. The method according to claim 21, **characterised in that**, updating the current reference baseline, comprises:
acquiring stable physiological information after the system error is over; and
updating the current reference baseline to the stable physiological information.

23. The method according to claim 21, **characterised in that**, the reference baseline setting interface is further operable to display the current reference baseline and/or the updated reference baseline.

24. The method according to claim 21, **characterised in that**, the reference baseline setting interface is further operable to display information for indicating whether there is the system error.

25. The method according to claim 16, **characterised in that**, after obtaining the physiological information of the target object, the method further comprises:
displaying an initial reference baseline setting interface when the physiological information is determined as a first valid value after the monitoring device is started; and
setting the first valid value as the reference baseline, if a confirmation instruction from a user that is used for setting the first valid value as the reference baseline, is received on the initial reference baseline setting interface; otherwise, setting a preset reference value as the reference baseline.

26. The method according to claim 16, **characterised in that**, after obtaining the physiological information of the target object, the method further comprises:
setting the physiological information as the reference baseline when the physiological information is determined as a first valid value after the monitoring device is started.

27. The method according to claim 16, **characterised in that**, the method further comprises:
displaying the analysis result of the monitoring device in real time.

28. The method according to claim 16, **characterised in that**, the state information includes least one of: connection position information between the target object and the signal acquisition apparatus, body position information of the target object, motion information of the target object and environment light source information.

29. The method according to claim 16, **characterised in that**, the physiological information includes ECG information, and the ECG information includes at least one of: QRS waveform shape, ST-T waveform shape, P-wave electrical axis and ST offset.

30. A computer-readable storage medium comprising a program that can be executed by a processor to implement the method according to any one of claims 16-29.
